# EUROPEAN PATENT APPLICATION

(11) **EP 1 264 897 A2**
(43) Date of publication of application: **11.12.2002**
(21) Application number: 01115483.8
(22) Date of filing: 27.06.2001
(51) Int. Cl.: C12Q 1/42, C12Q 1/48, G01N 33/58, C07K 7/06

(54) **Synthetic sensor peptide for kinase or phosphatase assays**

(30) Priority: 06.06.2001 EP 01113815
(71) Applicant: EUROPÄISCHES LABORATORIUM FÜR MOLEKULARBIOLOGIE (EMBL), D-69117 Heidelberg (DE)
(72) Inventor: Bastiaens, Philippe Igor, 69126 Heidelberg (DE)
(74) Representative: Böhm, Brigitte, Dipl.-Chem. Dr.

(57) **Abstract**

The present invention relates to a synthetic sensor peptide for the detection and/or measurement of a kinase or phosphatase activity of proteins wherein the synthetic sensor peptide serves as fluorescence resonance energy transfer (FRET) substrate undergoing a conformational change upon phosphorylation by said proteins which conformational change can be detected through FRET. Further, the present invention is concerned with a process for the qualitative and/or quantitative determination of proteins with kinase or phosphatase activity comprising said synthetic sensor peptide.

## Description

The present invention relates to a synthetic sensor peptide for the detection and/or measurement of a kinase or phosphatase activity of proteins wherein the synthetic sensor peptide serves as fluorescence resonance energy transfer (FRET) substrate undergoing a conformational change upon phosphorylation by said proteins which conformational change can be detected through FRET. Further, the present invention is concerned with a process for the qualitative and/or quantitative determination of proteins with kinase or phosphatase activity using said synthetic sensor peptide.

Protein kinases play a key role in the molecular mechanisms of signal transduction and catalyze phosphoryl group transfer reactions between ATP and specific serine, threonine and tyrosine residues of protein substrates. The kinases catalyze both the transfer of phosphoryl groups from ATP to phosphoryl acceptor proteins and the regeneration of ATP, i.e. the transfer of phosphoryl groups from phosphoryl donor proteins to ADP. ATP, which possesses a medium phosphoryl group transfer potential, thus functions as energy mediator between high-energy phosphoryl donor proteins and low-energy phosphoryl acceptor proteins in metabolism and signal transduction.

In contrast, phosphatases represent a group of enzymes catalyzing the hydrolysis of phosphoric acid esters in proteins. They participate in many metabolic reactions of the cell and function as antagonists for the kinases e.g. in signal transduction, wherein they dephosphorylate phosphorylated receptors, enzymes and other proteins with low phosphoryl group transfer potential.

The most convenient existing assays for kinases or phosphatases are based on the use of radioactive isotopes such as ³²P incorporated into (gamma-³²P) ATP. As it is necessary to apply radioactive isotopes, these kinase or phosphatase assays can not be performed in all conventional standard laboratories and are in addition very cost intensive.

The object of the present invention was to provide a phosphorylation assay that can be continuously followed in time, is technically simple and automatable, can be applied in live cells or organisms and is suitable for high-throughput screenings of chemical libraries.

This object is achieved according to the invention by a synthetic sensor peptide for the detection and/or measurement of a kinase or phosphatase activity of proteins comprising
(i) a kinase-specific phosphorylation site or a phosphatase-specific dephosphorylation site,
(ii) at least one linker containing at least one positively charged amino acid positioned at at least one end of the phosphorylation site or the dephosphorylation site and
(iii) a fluorescence resonance energy transfer (FRET) donor and acceptor fluorophore pair.

The concept of "using an" fluorescence energy transfer (FRET) substrate was first suggested by Carmel et al. (FEBS Letters 30, 11-14 (1973)) for an assay for proteases. In this approach a protease-specific substrate was designed with a donor fluorophore and an acceptor molecule which are positioned on either side of the bond to be cleaved by a protease and are sufficiently close that a large proportion of the fluorescence of the donor fluorophore is quenched by radiationless energy transfer to the acceptor molecule. Cleavage of the substrate by a protease then causes a large increase in distance of the donor fluorophore and the acceptor molecule which results in an increase in fluorescence. However, these FRET substrates, developed by Carmel et al., are limited to fluorophores that require UV excitation. This limits their use especially in compound screening methods. Furthermore, the donor fluorophore and the acceptor molecule are attached during synthesis of the FRET substrate. This generally requires specific reagents and know-how relating to their use. Accordingly, a range of donor and acceptor species can not be readily assessed for a given substrate.

WO94/2166 and WO97/28261 both provide novel FRET protease substrates for use in proteolytic assays and methods for their preparation, wherein after synthesis of the polypeptide substrate a donor or acceptor species is attached via the side chain of an amio acid comprised in the polypeptide substrate. This allows a wide range of donor and acceptor pairs to be attached to the polypeptide substrate and the selection of desirable donor and acceptor combinations.

The present invention makes use of the FRET concept as detection means for phosphorylation and dephosphorylation, respectively. The synthetic sensor peptide according to the invention is designed to be a specific substrate of a protein showing kinase or phosphatase activity. According to the invention, a synthetic sensor peptide serving as substrate for a specific kinase is phosphorylated at the kinase-specific phosphorylation site (i) by said kinase. Due to the negative charge of the transferred phosphoryl group the phosphorylated amino acid of the kinase-specific phosphorylation site (i) of the synthetic sensor peptide becomes negatively charged. Thereupon, the positively charged amino acids in the linkers (ii) at one or both ends of the kinase-specific phosphorylation site (i) are attracted to the negatively charged phosphate group of the phosphorylated amino acid causing a conformational change in the synthetic sensor peptide, which results in a change in distance of the donor and acceptor fluorophore molecules constituing the FRET donor and acceptor fluorophore pair (iii), which are positioned on opposite sides of the kinase-specific phosphorylation site (i). This causes a change in the FRET efficiency between the donor fluorophore molecule and the acceptor fluorophore molecule. On the one hand, the phosphorylation-induced conformational change in the synthetic sensor peptide can cause a large increase in distance between the donor fluorophore molecule and the acceptor fluorophore molecule which manifests itself by a decrease in FRET efficiency. On the other hand, the induced conformational change can also cause a large decrease in distance between the donor fluorophore molecule and the acceptor fluorophore molecule which manifests itself by an increase in FRET efficiency. However, a synthetic sensor peptide serving as substrate for a specific phosphatase is dephosphorylated at the phosphatase-specific dephosphorylation site (i) by said phosphatase. Thereby the attraction between the originally phosphorylated negatively charged amino acid of the phosphatase-specific dephosphorylation site (i) and the positively charged amino acids in the linkers (ii) at one or both ends of the phosphatase-specific dephosphorylation site (i) is neutralized. This causes a conformational change in the synthetic sensor peptide that results in a change in distance of the donor and acceptor fluorophore molecules constituing the FRET donor and acceptor fluorophore pair (iii) which are positioned on opposite sides of the phosphatase-specific dephosphorylation site (i). This causes a similar change in the FRET efficiency between the donor fluorophore molecule and the acceptor fluorophore molecule as mentioned above.

According to the invention, the change in the FRET efficiency between the donor fluorophore molecule and the acceptor fluorophore molecule of the synthetic sensor peptide can be detected either by a change in the fluorescence emission of the donor and acceptor fluorophore pair (iii) measured by ratiometric methods or a change in the fluorescence decay kinetics of the donor fluorophore molecule or the acceptor fluorophore molecule measured by time-resolved fluorescence techniques or fluorescence lifetime imaging.

In a preferred embodiment of the present invention the kinase-specific phosphorylation site or the phosphatase-specific dephosphorylation site (i) of the synthetic sensor peptide contains at least one amino acid. According to the invention, the amino acid of the kinase-specific phosphorylation site or the phosphatase-specific dephosphorylation site (i) is preferably serine, threonine, tyrosine, aspartate or histidine and serves as phosphorylation site or dephosporylation site for proteins with kinase or phosphatase activity. A person skilled in the art is able to easily design the sequence of the kinase-specific phosphorylation site or the phosphatase-specific dephosphorylation site (i) of the respective synthetic sensor peptide.

In a further embodiment of the present invention the kinase-specific phosphorylation site or the phosphatase-specific dephosphorylation site (i) further contains amino acids neighbouring the phosphorylation site or dephosphorylation site of a natural substrate of a protein with kinase or phosphatase activity of interest. Preferably, these amino acids comprise a specific kinase or phosphatase binding sequence or a respective consensus sequence that is recognized by a protein with kinase or phosphatase activity and mediates the interaction between the protein and the synthetic sensor peptide. Suitable amino acids can easily be determined by a man of ordinary skill.

Furthermore, the kinase-specific phosphorylation site or the phosphatase-specific dephosphorylation site (i) can comprise natural or synthetic amino acids joined by natural or modified peptide linkages.

In another preferred embodiment of the present invention the linkers (ii) of the synthetic sensor peptide are positioned at both ends of the kinase-specific phosphorylation site or the phosphatase-specific phosphorylation site (i). The sequence of the linkers (ii) containing at least one positively charged amino acid can be determined by a skilled artisan with the proviso that an attraction between the linker sequences and the negatively charged phosphate group which is transferred to the kinase-specific phosphorylation site (i) during phosphorylation of the synthetic sensor peptide by a kinase or loss of attraction by removal of the phosphatase group from the phosphatase-specific dephosphorylation site (i) induces a conformational change in the synthetic sensor peptide that can be detected via FRET.

It is further preferred that the linkers (ii) of the synthetic sensor peptide according to the invention contain 2 to 10 amino acids. The linkers (ii) contain natural or synthetic positively charged amino acids joined by natural or modified peptide linkages. Suitable natural positively charged amino acids are in particular arginine, histidine and lysine.

The FRET donor and acceptor fluorophore pair (iii) of the synthetic sensor peptide according to the invention comprises a donor fluorophore molecule and a acceptor fluorophore molecule. The donor and acceptor fluorophore molecules are located on opposite sides of the kinase-specific phosphorylation site or the phosphatase-specific dephosphorylation site (i) and the linker(s) (ii), respectively. The positions of the donor fluorophore molecule and the acceptor fluorophore molecule within the synthetic sensor peptide can be selected freely within these ranges with the proviso that the phosphorylation or dephosphorylation of the synthetic sensor peptide is not affected to any significant extent and that the conformational change of the synthetic sensor peptide induced by phosphorylation or dephosphorylation results in as large an increase or decrease in distance of the donor and acceptor fluorophore molecules as possible. However, the distance between both fluorophore molecules is selected so that the synthetic sensor peptide can exhibit fluorescence resonance energy transfer (FRET). Namely, the donor fluorophore molecule is excited by light of appropriate intensity within the donor's excitation spectrum. The donor fluorophore molecule then emits the absorbed energy as fluorescent light. If the donor and acceptor fluorophore molecules are sufficiantly close, the fluorescent energy is quenched by the acceptor fluorophore molecule. If the donor and acceptor fluorophore molecules are far apart, on the other hand, fluorescence energy is not or only partly quenched by the acceptor fluorophore molecule. FRET is defined as a reduction in the intensity of the fluorescent signal from the donor fluorophore molecule, as a reduction in the lifetime of excited state and/or as re-emission of fluorescent light at lower energy levels characteristic for the acceptor fluorophore molecule. When the conformational change of the synthetic sensor peptide results in an increase in distance of the donor and acceptor fluorophore molecules, FRET is diminished or eliminated. On the other hand a decrease in distance of both fluorophore molecules upon phosphorylation or dephosphorylation causes an increase in FRET.

Any FRET donor and acceptor fluorophore pair may be used in the invention, including as preferably used pairs oregon green/tetramethyl rhodamine, fluoresceine/rhodamine, Cy3/Cy5, Cy2/Cy3, Alexa 488/Alexa 543. However, the donor and acceptor fluorophore molecules of the FRET donor and acceptor fluorophore pair (iii) are selected so that the donor and acceptor fluorophore molecules exhibit FRET when the donor fluorophore molecule is excited. Therefore at least one factor to be considered in chosing the FRET donor and acceptor fluorophore pair (iii) is the efficiency of FRET between them. Additional factors, that may be considered by a skilled artisan in the selection of the donor and acceptor fluorophore pair include, among other things, the degree of energy transfer, which determines the initial level of fluorescence of the synthetic sensor peptide, the excitation and emission wavelengths of the donor and acceptor fluorophore molecules and the level of substrate activity of the synthetic sensor peptide.

The total length of the peptide chain of the synthetic sensor peptide according to the invention is preferably in the range of 6 to 45 amino acids, more preferably 6 to 30 amino acids and most preferably 6 to 15 amino acids. The invention, preferably, also provides that the synthetic sensor peptide according to the invention has one of the peptide sequences shown in SEQ ID NOs:1-6.

In a preferred embodiment of the present invention the synthetic sensor peptide is designed to be cell permeable. In this case additional amino acid sequences can be added at one or both ends of the synthetic sensor peptide to mediate cell permeability. Preferably, these additional sequences are signal sequences that serve to import the peptide into cellular compartments such as the endoplasmic reticulum, mitochondria, peroxisomes or even the nucleus. The appropriate signal sequences for these cellular compartments are known in the art and will be apparent to a person of ordinary skill. Another way of making the synthetic sensor peptide according to the invention cell permeable is by using an acetoxy methyl ester derivative of one of the two fluorophore molecules such as fluorescein which then has to be positioned at one end of the synthetic sensor peptide. This transports the peptide into cells where esterases release acetic acid and methanal thereby loading the fluorophore derivative into cells. This non-invasive and technically straightforward acetoxymethyl ester technique is by far the most popular method for loading fluorescent indicators into cells. The carboxylate groups or the phenolic hydroxyl groups of indicators are derivatized as acetoxymethyl or acetate esters, respectively, rendering the indicator permeant to membranes. Once inside the cell, these derivatized indicators are hydrolyzed by intracellular esterases, releasing the polyanionic indicator (R.Y. Tsien (1980), A non-disruptive technique for loading calcium buffers and indicators into cells, Nature 290, 527).

The synthetic sensor peptide according to the invention is prepared according to standard techniques used for synthetic peptide synthesis. The techniques and procedures are generally performed according to conventional methods in the art and as described in various general references (such as Sambrook et al., Molecular Cloning: A Laboratory Manual, 2nd Edition (1989), Cold Spring Harbor, Laboratory Press, Cold Spring Harbor, New York). According to the present invention, the synthetic sensor peptide can be expressed recombinantly in prokaryotic or eukaryotic cells or can be prepared by direct protein synthesis. However, the synthetic sensor peptide according to the invention is conveniently prepared by direct synthesis (cf. Solid Phase Peptide Synthesis, E. Atherton and R.C. Sheppard, IRL Press (1989)). The invention further provides that the FRET donor and acceptor fluorophore pair (iii) is coupled to the synthetic sensor peptide during synthesis using modified amino acids linked to the appropriate donor and acceptor fluorophore molecules or by subsequent reaction of the peptide part of the synthetic sensor peptide after synthesis with the reactive donor and acceptor fluorophore molecules of the appropriate FRET donor and acceptor fluorophore pair (iii). Preferably, the donor and acceptor fluorophore molecules are attached via a side chain of an amino acid of the synthetic sensor peptide. The sites for attachment of the reactive donor and acceptor fluorophore molecules are preferably provided by amino groups and thiol groups of the amino acids cysteine, lysine, arginine or the N-terminal amino acid. Convenient donor and acceptor fluorophore molecules for reaction at thiol groups or amino groups of said amino acids include donor and acceptor fluorophore molecules derivatized with maleimido groups, haloacetyl groups and isothiocyanates, active esters of carboxylic acids, sulfonyl halides, respecively. The reaction takes place at moderate pH and temperature conditions, conventiently in stoichiometric amounts. The purification of the synthetic sensor peptide or the derivatized amino acids can be achieved e.g. by gel permeation chromatography, HPLC or by other methods known to the person of ordinary skill. The invention further provides that the synthetic sensor peptide can also be produced according to processes as described in WO94/2166 and WO97/28261.

A further subject matter of the present invention is a process for the qualitative and/or quantitative determination of proteins with kinase or phosphatase activity characterized in that a sample to be assayed is contacted with the synthetic sensor peptide according to the invention and changes in the fluorescence or fluorescence kinetics of the sensor are monitored. According to the present invention, a sample can be a living cell or organism, a protein preparation and a cell homogenate.

The process according to the invention is based on fluorescence resonance energy transfer (FRET) and especially on changes in the FRET efficiency between the donor and acceptor fluorophore molecules of the synthetic sensor peptide described above. Fluorescence is measured preferably but not limited to by ratiometric dual wavelength fluorescence detection, or fluorescence lifetime determination of the donor or acceptor fluorophore molecule of the synthetic sensor peptide according to the invention. Fluorescence measurement methods are known to those skilled in the art and are described e.g. in Lakowicz, J.R., Principles of Fluorescence Spectroscopy, Plenum Press, New York, (1983).

In a preferred embodiment of the present invention a change in fluorescence emission of the donor fluorophore molecule is detected or a change in the fluorescence decay kinetics of the donor fluorophore molecule or the acceptor fluorophore molecule is detected. When detecting the changes in FRET efficiency by a change in the fluorescence emission of the donor fluorophore molecule, the donor and acceptor fluorophore molecule are initially internally calibrated by ratiometric methods. A change in the fluorescence decay kinetics of the donor fluorophore molecule or the acceptor fluorophore molecule is preferably measured by time-resolved fluorescence techniques or fluorescence lifetime imaging.

The process according to the invention can either be performed as an in vivo assay, wherein the sample to be tested is a living cell or an organism or as an in vitro assay, wherein the sample to be tested is a protein preparation or a cell homogenate. When performing the enzymatic assay on living cells in vivo, the synthetic sensor peptide can either be expressed recombinantly inside the cells by using donor and acceptor fluorescence molecule derivatized amino acids or can be introduced into a cell or cellular compartment as described above. The amount of enzyme activity in the cell or cellular compartment is determined by measuring changes in FRET between the donor and acceptor fluorophore molecules in the cells. If the cell or cellular compartment does not contain any kinase or phosphatase activity, the FRET efficiency in the cell is constant. If the cell, however, possesses a high degree of kinase or phosphatase activity most of the synthetic sensor peptide molecules are phosphorylated or dephosphorylated and therefore undergo a conformational change inducing a change in the FRET efficiency. A large increase or decrease in FRET efficiency reflects a large amount or high efficiency of proteins with kinase or phosphatase activity.

By using a synthetic sensor peptide according to the invention, a fluorescence assay is provided for the determination of the kinase activity or phosphatase activity of a protein or protein preparation or cellular sample in vitro or in vivo. The process according to the invention is the first description of a synthetic sensor peptide based phosphorylation or dephosphorylation assay that makes use of FRET to measure a conformational change in the synthetic sensor peptide after phosphorylation or dephosphorylation. The process according to the invention can be used in any conventional laboratory due to its broad applicability, its sensitivity and specificity.

A further subject of the present invention is the use of a process according to the invention for high-throughput screenings of drug libraries. Preferably, the process according to the invention can be used in pharmacological industry for high-throughput screening of drug libraries, e.g. to identify compounds that alter the activity of an enzyme. Preferably the assay is then performed as an in vitro assay on a sample containing a known amount of the enzyme, the protein-specific synthetic sensor peptide and a test compound selected from a drug library. The amount of the enzyme activity in the sample is then determined as described above and the amount of activity per mole of enzyme in the presence of the test compound is compared with the activity per mole of enzyme in the absence of the test compound. A difference in enzymatic activity indicates that the test compound alters the activity of the enzyme. The invention, however, also provides that this drug screening assay is performed in vivo.

A still further subject of the present invention is the use of a process according to the invention for target validation. Preferably, the process according to the invention can be used for monitoring the activity of a gene product in order to establish its activity or participation in a biological pathway or process.

The invention is further illustrated by the following figures and examples:

### Figures

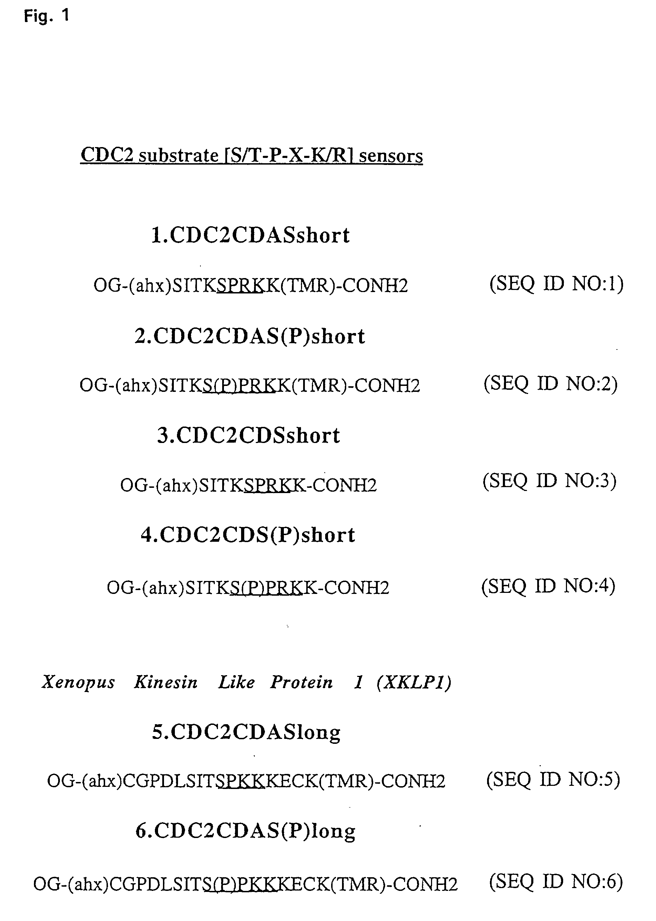

**Figure 1** shows the sequences SEQ ID NOs:1-6 of synthetic sensor peptides according to the invention: P denotes peptides synthesized with a phosphoserine. ahx stands for aminohexanoic acid spacer, CONH2 represents the C-terminal amide, OG is oregon green 488 and TMR is tetramethyl rhodamine (5-carbonyl).

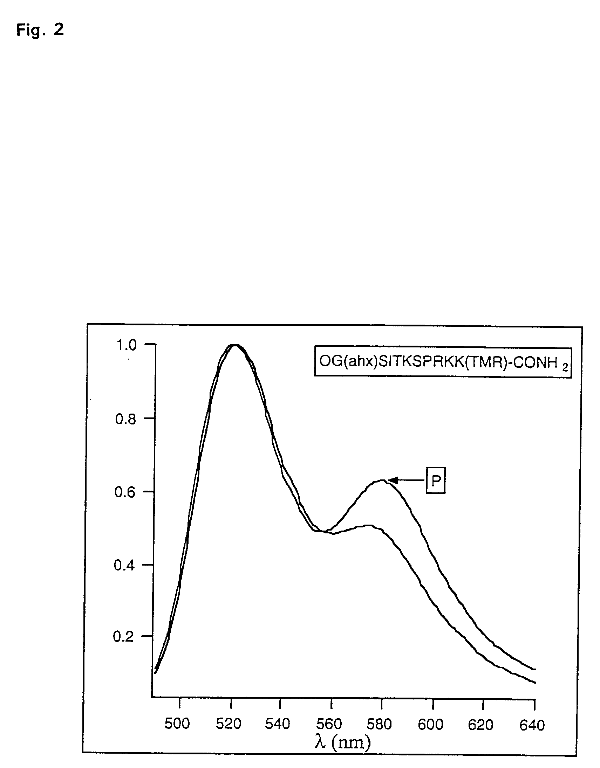

**Figure 2** shows a fluorescence emission spectra of a synthentic sensor peptide according to the invention (OG(ahx)SITKSPRKK(TMR)-CONH₂ (SEQ ID NO:1)) prepared in order to measure the kinase activity of the protein CDC2. The lower emission curve shows the FRET efficiency of the unphosphorylated synthetic sensor peptide. The upper emission curve indicated by P shows the FRET efficiency measured in the phosphorylated synthetic sensor peptide. The FRET efficiency between the donor fluorophore molecule and the acceptor fluorophore molecule of the synthetic sensor peptide increases upon phosphorylation as seen from the increased sensitized emission at 580 nm. The short peptide was dissolved in phosphate buffered saline at 0.87 micromolar, and the phosphorylated peptide was dissolved in phosphate buffered saline at 0.21 micromolar.

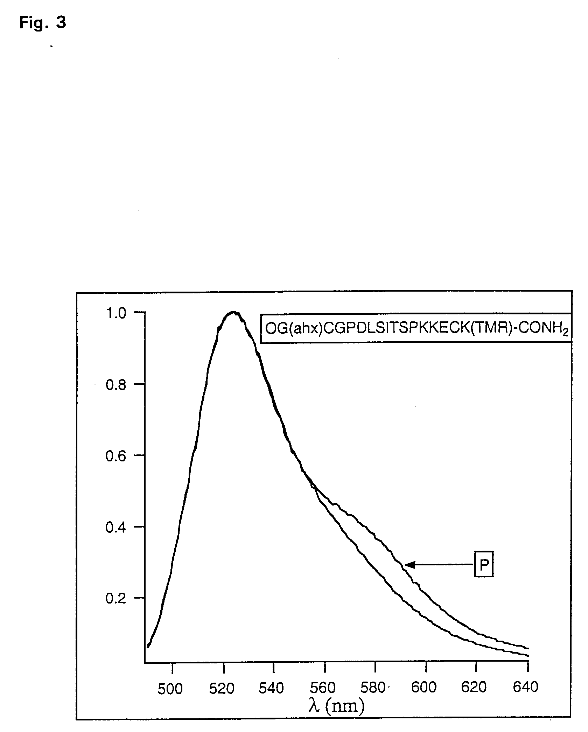

**Figure 3** shows a fluorescence emission spectra of a synthentic sensor peptide according to the invention (OG(ahx)GGPDLSITSPKKECK(TMR)-ONH₂ (SEQ ID NO:5)) prepared in order to measure the kinase activity of the protein CDC2. The lower emission curve shows the FRET efficiency of the unphosphorylated synthetic sensor peptide. The upper emission curve indicated by P shows the FRET efficiency measured in the phosphorylated synthetic sensor peptide. The FRET efficiency between the donor fluorophore molecule and the acceptor fluorophore molecule of the synthetic sensor peptide increases upon phosphorylation as seen from the increased sensitized emission at 580 nm. The long peptide was dissolved in phosphate buffered saline at 0.55 micromolar, and the phosphorylated long peptide was dissolved in phosphate buffered saline at 0.45 micromolar.

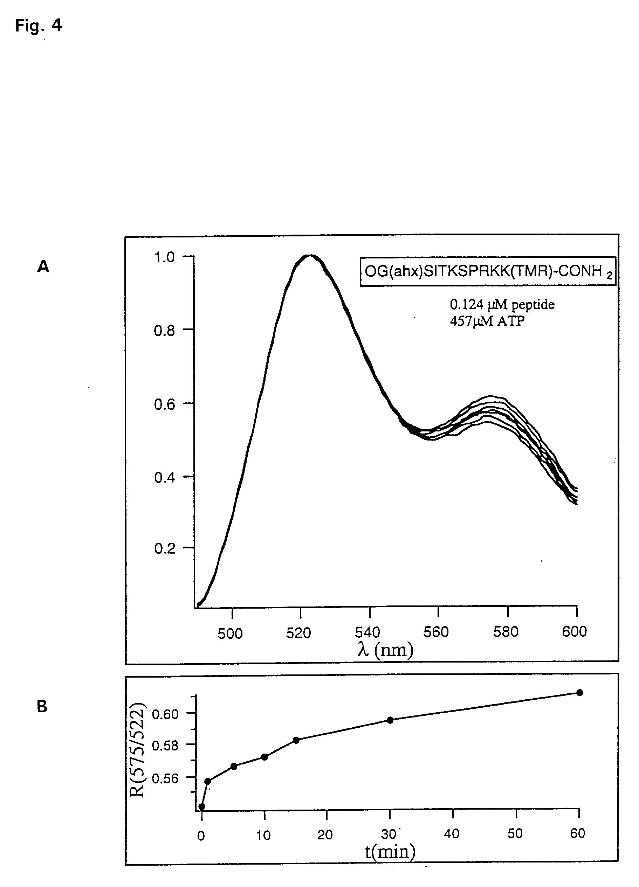

**Figure 4** shows the results of a CDC2 kinase activity assay according to the invention using the synthetic sensor peptide (OG(ahx)SITKSPRKK (TMR)-CONH₂ (SEQ ID NO:1)). To 300 microliter buffer (80 mM beta-glycerophosphate, pH 7.3, 20 mM EGTA, 15 mM MgCl₂) was added 40 microliter 4 mM ATP (final concentration of ATP in reaction mixture: 0.457 mM) and 5 microliter peptide stock solution (8.7 micromolar peptide in 80 mM beta-glyerophosphate, pH 7.3, 20 mM EGTA, 15 mM MgCl₂; final concentration of peptide in reaction mixture: 0.12 micromolar). The reaction was started by adding 5 microliter of purified CDC2/cyclinB with a specific activity of 100 pmole/min/microliter. Note that the reaction is slower as expected from the specific activity since the peptide was at a concentration much below Vmax conditions (Km for CDC2 substrate is 40 micromolar). **A** shows the fluorescence emission changes of the synthetic sensor peptide over time after addition of CDC2 to the sample due to an increase in FRET efficiency upon phosyphorylation. **B** shows the fluorescence intensities ratio of acceptor fluorophore molecule/donor fluorophore molecule plotted as a function of time.

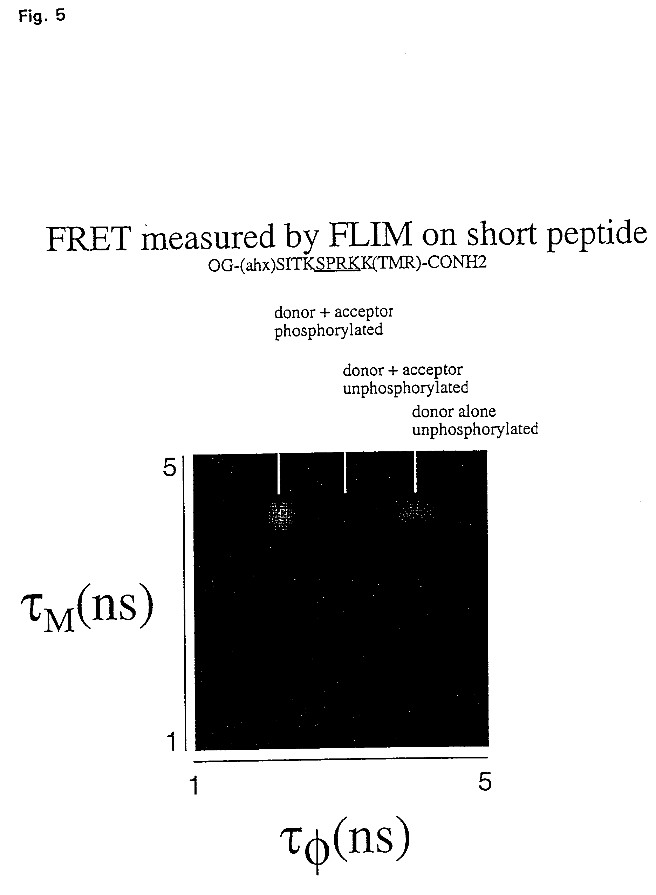

**Figure 5** shows the two-dimensional histogram of phase and modulation lifetimes measured by FLIM on the synthetic sensor peptide (OG(ahx)SITKSPRKK(TMR)-CONH₂ (SEQ ID NO:1)). The fluorescence lifetimes were measured with a frequency domain FLIM system at 80 MHz excitation frequency (see Squire and Bastiaens (1999), Three dimensional image restoration in fluorescence lifetime imaging microscopy. J. Microsc., 193, 36-49). Measurements were taken on 20 microliter droplets of the dissolved peptide in a humidified glass bottomed chamber. The determined phase and modulation lifetimes are represented on the two axes of the histogram. The short peptide was dissolved in phosphate buffered saline at 0.87 micromolar, and the phosphorylated peptide was dissolved in phosphate buffered saline at 0.21 micromolar. The fluorescence lifetime can be seen for the donor fluorophore molecule alone (unphosphorylated), the donor fluorophore molecule and acceptor fluorophore molecule (unphosphorylated) and donor fluorophore molecule and acceptor fluorophore molecule (phosphorylated). The smaller fluorescence lifetime of the phosphorylated peptide as compared to the unphosphorylated peptide shows an increase in FRET efficiency upon phosphorylation.

## Claims

1. Synthetic sensor peptide for the detection and/or measurement of a kinase or phosphatase activity of proteins comprising
(i) a kinase-specific phosphorylation site or a phosphatase-specific dephosphorylation site,
(ii) at least one linker containing at least one positively charged amino acid positioned at at least one end of the kinase-specific phosphorylation site or the phosphatase-specific dephosphorylation site and
(iii) a fluorescence resonance energy transfer (FRET) donor and acceptor fluorophore pair.

2. Synthetic sensor peptide according to claim 1,
wherein the kinase-specific phosphorylation site or the phosphatase-specific dephosphorylation site (i) contains at least 1 amino acid.

3. Synthetic sensor peptide according to claims 1 or 2,
wherein the kinase-specific phosphorylation site or the phosphatase-specific dephosphorylation site (i) contains further amino acids neighbouring the phosphorylation site of a kinase substrate or the dephosphorylation site of a phosphatase substrate.

4. Synthetic sensor peptide according to any one of claims 1 to 3,
wherein linkers (ii) are contained at both ends of the kinase-specific phosphorylation site or the phosphatase-specific dephosphorylation site.

5. Synthetic sensor peptide according to any one of claims 1 to 4,
wherein linkers (ii) contain 2 to 10 amino acids.

6. Synthetic sensor peptide according to any one of claims 1 to 5,
wherein linkers (ii) contain natural or synthetic positively charged amino acids.

7. Synthetic sensor peptide according to any one of claims 1 to 6,
wherein the FRET donor and acceptor fluorophore pair (iii) is oregon green/tetramethyl rhodamine, fluorescein/rhodamine, Cy3/Cy5, Cy2/Cy3, Alexa 488/Alexa 543.

8. Synthetic sensor peptide according to any one of claims 1 to 7,
wherein it is cell permeable.

9. Synthetic sensor peptide according to any one of claims 1 to 7,
wherein it has one of the sequences shown in SEQ ID NOs:1-6.

10. Process for the qualitative and/or quantitative determination of proteins with kinase or phosphatase activity
**characterized in that** a sample to be assayed is contacted with a synthetic sensor peptide according to any one of claims 1 to 9 and changes in the fluorescence or fluorescence kinetics of the sensor are monitored.

11. Process according to claim 10,
wherein the fluorescence is measured by ratiometric dual wavelength fluorescence detection or fluorescence lifetime determination of the fluorescent donor.

12. Process according to claims 10 or 11,
wherein a change in fluorescence emission of the donor fluorophore molecule is detected or a change in the fluorescence decay kinetics of the donor fluorophore molecule or the acceptor fluorophore molecule is detected.

13. Process according to any one of claims 10 to 12,
wherein the process is conducted as an in vitro assay and the sample is a protein preparation or cell homogenate.

14. Process according to any one of claims 10 to 13,
wherein it is an in vivo assay and the sample is a living cell or an organism.

15. Use of a process according to claims 10-14 for high-throughput screenings of drug libraries.

16. Use of a process according to claims 10-14 for target validation.
